# EUROPÄISCHE PATENTSCHRIFT

(11) **EP 0 395 938 B1**
(45) Veröffentlichungstag und Bekanntmachung des Hinweises auf die Patenterteilung: **17.01.1996**
(21) Anmeldenummer: 90107370.0
(22) Anmeldetag: 18.04.1990
(51) Int. Cl.: C07D 251/24

(54) **Verfahren zur Herstellung von 2-(2',4'-Dihydroxyphenyl)-4,6-diaryl-s-triazinen**
Process for the preparation of 2-(2',4'-dihydroxyphenyl)-4,6-diaryl-s-triazines
Procédé pour la préparation de 2-(2',4'-dihydroxyphényl)-4,6-diaryl-s-triazines

(30) Priorität: 21.04.1989 CH 1536/89
(43) Veröffentlichungstag der Anmeldung: 07.11.1990
(73) Patentinhaber: CIBA-GEIGY AG, CH-4002 Basel (CH)
(72) Erfinder: Burdeska, Kurt, Dr., CH-4058 Basel (CH); Günter, Franz, Dr., CH-4125 Riehen (CH)
(74) Vertreter: Zumstein, Fritz, Dr.

(56) Entgegenhaltungen:
- CH-A- 484 695
- HELVETICA CHIMICA ACTA, Band 55, Nr. 1, 1972, Seiten 1566-1595, Basel, CH; H. BRUNETTI et al.: "Die Synthese von asymmetrisch substituierten o-Hydroxyphenyl- s-triazinen"
- CHEMISCHE BERICHTE, Band 100, 1967, Seiten 1874-1891, Weinheim, DE; H. EILINGSFELD et al.: "Synthese von 1.3.5-Triazinen"

## Beschreibung

Die vorliegende Erfindung betrifft ein neues Verfahren zur Herstellung von 2-(2',4'-Dihydroxyphenyl)-4,6-diaryl-s-triazinen.

Asymmetrisch substituierte Dihydroxyphenyl-s-triazine sind z.B. aus der US-A-3,268,474 bekannt. Man gelangt zu diesen Verbindungen, indem man zunächst 1 Mol Cyanurchlorid in Gegenwart eines Friedel-Crafts-Katalysators mit 2 Mol Dimethylbenzol und das erhaltene Monochloraryltriazin in einer weiteren Friedel-Crafts-Reaktion mit Dihydroxybenzol umsetzt. Als Lösungsmittel für diese Reaktion dienen Nitrobenzol, o-Dichlorbenzol (oDCB), Chlorbenzol oder hochchlorierte Lösungsmittel. Die Friedel-Crafts-Reaktion von s-Trichlortriazin mit aromatischen Kohlenwasserstoffen verläuft allerdings wenig selektiv. Neben den Monochlor-diaryltriazinen entstehen schier trennbare Gemische von Dichloraryltriazinen und Triaryltriazinen (Helv. 55, 1589 (1972)).

Es wurde nun ein Verfahren gefunden, welches die Herstellung von 2-(2',4'-Dihydroxyphenyl)-4,6-diaryl-s-triazinen in einfacher Weise und in sehr guten Ausbeuten ermöglicht.

Das erfindungsgemässe Verfahren zur Herstellung von 2-(2',4'-Dihydroxyphenyl)-4,6-diaryl-s-triazinen der Formel 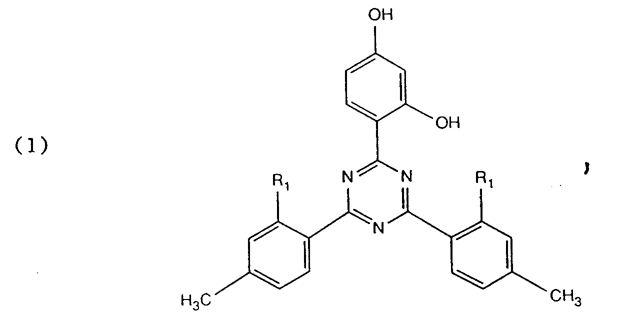
wobei R₁ Methyl oder Wasserstoff bedeutet, durch Substitution der Methylthiogruppe einer Methylthio-diaryl-s-triazin-Verbindung der Formel 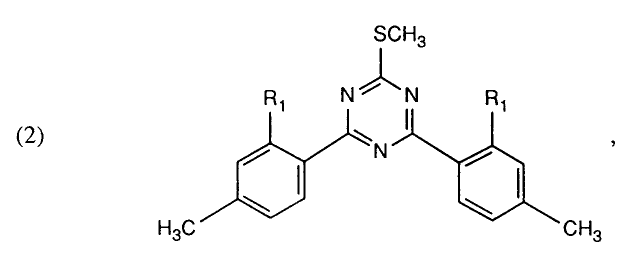
wobei R₁ die oben angegebene Bedeutung hat, durch ein Chloratom und durch Umsetzung der erhaltenen Verbindung der Formel 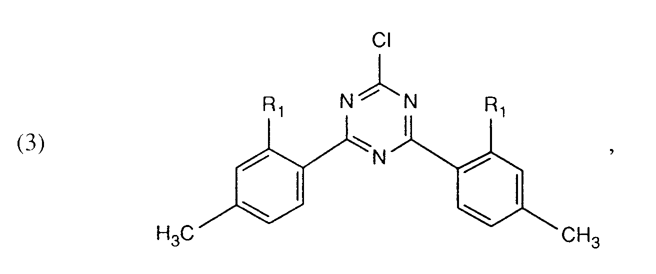
wobei R₁ die oben angegebene Bedeutung hat, mit 1,3-Dihydroxybenzol mittels einer Lewis-Säure zu einer Verbindung der Formel (1) ist dadurch gekennzeichnet, dass man in einer ersten Stufe die Verbindung der Formel 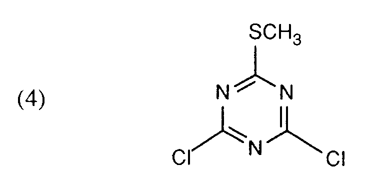
mittels einer Lewis-Säure mit m-Xylol oder Toluol umsetzt, in einer zweiten Stufe die erhaltene Verbindung der Formel (2) in Gegenwart von m-Xylol oder Toluol mit Chlor oder Sulfurylchlorid zur Verbindung der Formel (3) umsetzt und in einer dritten Stufe die erhaltene Verbindung mit 1,3-Dihydroxybenzol mittels einer Lewis-Säure zur Verbindung der Formel (1) in Gegenwart von Toluol, m-Xylol oder Xylol-Isomerengemisch umsetzt.

Unter "Aryl" wird in der vorliegenden Beschreibung Tolyl oder Xylyl verstanden.

Zur Herstellung der Verbindungen der Formeln (1) und (2) wird als Lewis-Säure vorzugsweise Aluminiumchlorid eingesetzt.

Die Diarylmonochlortriazinverbindung der Formel (3) kann auch aus der Verbindung (4) ohne Isolierung der Methylthiostufe der Formel (2) in einem Eintopf-Prozess hergestellt werden.

Die Reaktionstemperatur der einzelnen Stufen kann in weiten Grenzen variieren, z.B. zwischen 30 und 100°C. Die Reaktion der ersten Stufe wird zwischen 55 und 90°C, die der zweiten Stufe zwischen 0 und 80°C und die der dritten Stufe zwischen 60 und 100°C durchgeführt.

Bevorzugte Reaktionstemperaturen liegen bei der ersten Stufe zwischen 65 und 75°C, bei der zweiten Stufe zwischen 40 und 50°C und bei der dritten Stufe zwischen 80 und 90°C.

Die Herstellung der Ausgangsverbindung gemäss Formel (4) erfolgt durch Umsetzung von Cyanurchlorid mit Methylmercaptan. Diese Reaktion ist ausführlich beschrieben in Rec. Trav. chim. Pays Bas 78, 967 (1959).

Die Verwendung von Dichlormethylthio-s-triazin anstelle der entsprechenden Methoxy-Verbindung hat den Vorteil, dass bei der Friedel-Crafts-Reaktion mit dem substituierten Benzol entsprechend Formel (5) selektiv das Diaryl-s-triazin entsprechend Formel (2) gebildet wird. Bei der entsprechenden Friedel-Crafts-Reaktion von Dichlormethoxy-s-triazin findet hingegen stets eine Entalkylierung der Methoxygruppe statt (Helv. 55, 1575 (1972)).

Die nach dem erfindungsgemässen Verfahren in der ersten Stufe hergestellte Methylthio-s-triazin-Verbindung der Formel (2), worin R₁ Methyl ist, stellt eine neue Verbindung dar.

Die Reaktion der zweiten Stufe, bei der die Methylthiogruppe der Verbindungen der Formel (2) durch ein Chloratom ausgetauscht wird, ist in Chem. Ber. 100, 1874 (1967) beschrieben. Während in dieser Publikation die Reaktion in Tetrachlorkohlenstoff durchgeführt wird, erfolgt das erfindungsgemässe Verfahren in Toluol oder Xylol.

Die bei der Reaktion der zweiten Stufe gebildeten Verbindungen der Formel (3) sind ebenfalls aus Helv. 55, 1589 (1972) bekannt.

Die Friedel-Crafts-Reaktion von Monochlordiphenyltriazinen mit Dihydroxybenzol, die der Reaktion der dritten Stufe entspricht, ist in zahlreichen Literaturstellen beschrieben, wie beispielsweise in der US-A-3,268,474, jedoch wird die Reaktion bei allen Referenzen in Nitrobenzol, oDCB oder Chlorbenzol durchgeführt, während beim erfindungsgemässen Verfahren diese Reaktion stets in Toluol, Xylol oder Xylol-Isomerengemischen durchgeführt wird, ohne dass eine Reaktion mit dem Lösungsmittel erfolgt, was sehr überraschend ist.

Das erfindungsgemässe Verfahren beschreibt einen neuen Weg zur Synthese von asymmetrischen Dihydroxyphenyl-s-triazinen, wobei sämtliche Reaktionsstufen in einem einzigen organischen Lösungsmittel durchgeführt werden können und dabei hohe Ausbeuten erzielt werden.

Die nach dem erfindungsgemässen Verfahren hergestellten Verbindungen finden Verwendung als UV-Absorber oder sind Ausgangsprodukte für die Herstellung von UV-Absorbern.

Die folgenden Beispiele dienen zur Erläuterung der Erfindung.

### Beispiel 1:

293 g wasserfreies Aluminiumchlorid (sublimiert, Fa. Merck) werden in 600 ml Toluol suspendiert und auf 70-75°C erhitzt. In die Suspension lässt man unter Rühren während 1,5 Stunden eine Lösung von 200,1 g 2,4-Dichlor-6-methylthio-1,3,5-triazin in 550 ml Toluol einfliessen. Die Temperatur soll dabei 80°C nicht übersteigen. Anschliessend wird auf 85-90°C erhitzt und noch 5,5 Stunden bei dieser Temperatur gerührt. Man lässt danach auf 50°C abkühlen und rührt das Gemisch in 600 ml Wasser und 150 ml 30%iger Salzsäure unter Kühlung so ein, dass die Temperatur zwischen 50-60°C bleibt. Danach wird unter Rückfluss erhitzt und das Toluol mit Wasserdampf abdestilliert. Das Produkt wird noch warm abfiltriert mit heissem Wasser, dann mit Methanol gewaschen und getrocknet. Man erhält 280 g (91,2 % der Theorie) der Verbindung der Formel 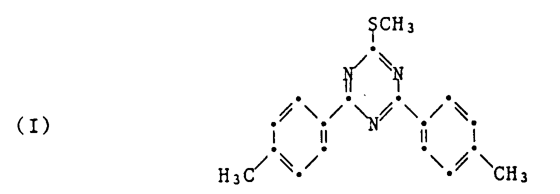
vom Schmelzpunkt 161-163°C. Verwendet man anstelle der 600 ml Toluol 600 ml m-Xylol, erhält man bei sonst gleicher Arbeitsweise die Verbindung der Formel 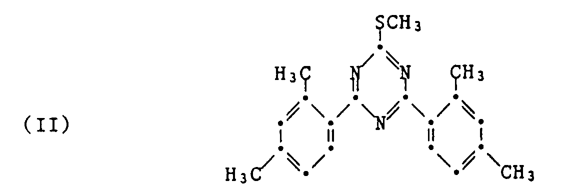
vom Schmelzpunkt 82-83°C.

### Beispiel 2:

280 g Aluminiumchlorid wasserfrei (sublimiert Fa. Merck) werden in 600 ml m-Xylol suspendiert und auf 65-70°C erhitzt. In die Suspension lässt man danach innerhalb von 1,5 Stunden bei 65-70°C unter gutem Rühren eine Lösung von 200,1 g 2,4-Dichlor-6-methylthio-1,3,5-triazin (98 %) in 420 ml m-Xylol einfliessen. Anschliessend wird zur Beendigung der Reaktion noch 1,5 Stunden bei 65-70°C nachgerührt. Danach lässt man das Reaktionsgemisch auf 50°C abkühlen und rührt es dann in ein Gemisch aus 900 ml Wasser und 100 ml 30%iger Salzsäure ein. Die Temperatur soll dabei 80°C nicht übersteigen. Nach weiterem 10minütigem Rühren wird die wässrige Phase von der Xylolschicht abgetrennt. In die Xylolschicht, enthaltend das 2-Methylthio-4,6-bis(2′,4′-dimethylphenyl)-1,3,5-triazin, leitet man nach dem Abkühlen auf 35-40°C innerhalb von zwei Stunden 140 g Chlor ein, wobei das 2-Chlor-4,6-bis(2′,4′-dimethylphenyl)-1,3,5-triazin ausfällt. Es wird bei 0-5°C abfiltriert, mit Xylol und Petrolether gewaschen und getrocknet. Man erhält 266 g (82,1 % der Theorie) der Verbindung der Formel 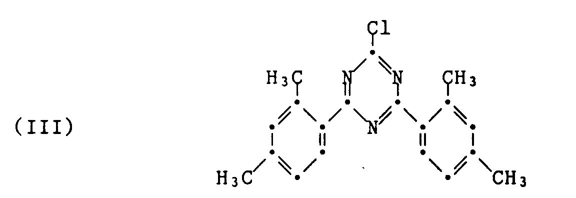
vom Schmelzpunkt 134-136°C.

Die anstelle von m-Xylol mit Toluol auf analogem Weg hergestellte Verbindung der Formel 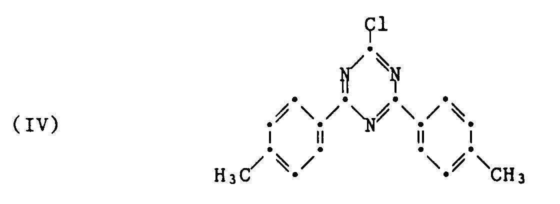
hat einen Schmelzpunkt von 204-206°C.

### Beispiel 3:

Die Verbindung der Formel (IV) kann auch auf folgendem Weg hergestellt werden:

30,7 g 2-Methylthio-4,6-bis(4′-methylphenyl)-1,3,5-triazin werden in 70 ml Toluol suspendiert und auf 55 bis 60°C erhitzt. Unter gutem Rühren lässt man danach eine Lösung von 27 g Sulfurylchlorid in 20 ml Toluol innerhalb von 40 Minuten zufliessen. Danach wird noch 30 Minuten bei 60°C nachgerührt, anschliessend auf 5°C abgekühlt und das ausgefallene Produkt abfiltriert. Es wird mit Toluol gewaschen und bei 80°C im Vakuum getrocknet.
Ausbeute: 25,3 g
Das Produkt hat einen Schmelzpunkt von 204-206°C.

### Beispiel 4:

In einem Xylol-Isomerengemisch von 400 ml werden nacheinander 161,9 g 2-Chlor-4,6-bis(2′,4′-dimethylphenyl)-s-triazin und 73,4 g wasserfreies Aluminiumchlorid eingetragen. Die Suspension wird auf 70-75°C erhitzt. Bei dieser Temperatur lässt man eine Suspension von 66 g Resorcin und einem Xylol-Isomerengemisch von 100 ml innerhalb von 1,5 Stunden zufliessen. Die Suspension wird während einer Stunde auf 85-90°C erhitzt und 3 Stunden bei dieser Temperatur gerührt. Nach dem Abkühlen auf 60°C lässt man das Reaktionsgemisch in 400 ml Wasser und 100 ml 30%iger Salzsäure einfliessen. Das nach Abdestillation des Xylol-Isomerengemisches angefallene gelbe Produkt wird noch heiss filtriert, mit heissem Wasser und Methanol gewaschen und getrocknet. Die Ausbeute beträgt 175-177 g (88-89 % der Theorie) der Verbindung der Formel 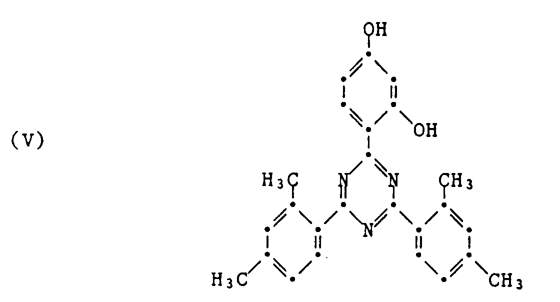
vom Schmelzpunkt 199-201°C.

Die anstelle von m-Xylol mit Toluol auf analogem Weg hergestellte Verbindung der Formel 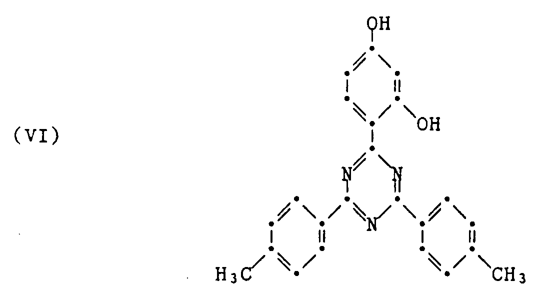
hat einen Schmelzpunkt von 292 bis 293°C.

## Patentansprüche

1. Verfahren zur Herstellung von 2-(2',4'-Dihydroxyphenyl)-4,6-diaryl-s-triazinen der Formel 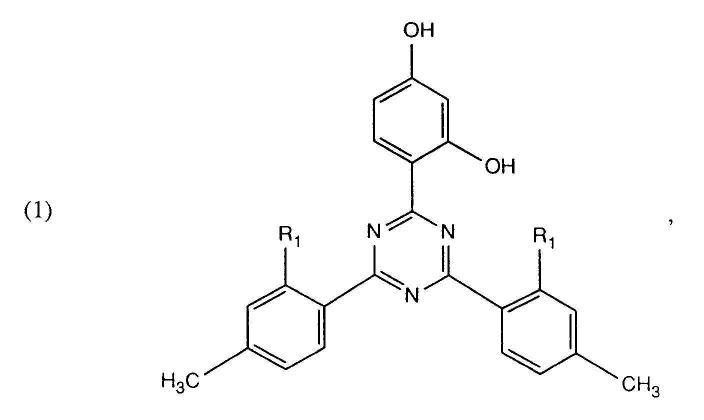 wobei R₁ Methyl oder Wasserstoff bedeutet, durch Substitution der Methylthiogruppe einer Methylthio-diaryl-s-triazin-Verbindung der Formel 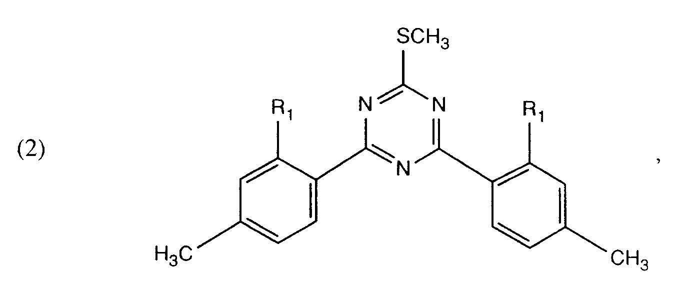 wobei R₁ die oben angegebene Bedeutung hat, durch ein Chloratom und durch Umsetzung der erhaltenen Verbindung der Formel 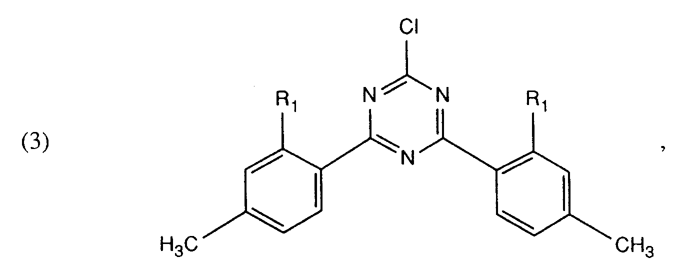 wobei R₁ die oben angegebene Bedeutung hat, mit 1,3-Dihydroxybenzol mittels einer Lewis-Säure zu einer Verbindung der Formel (1), dadurch gekennzeichnet, dass man in einer ersten Stufe die Verbindung der Formel 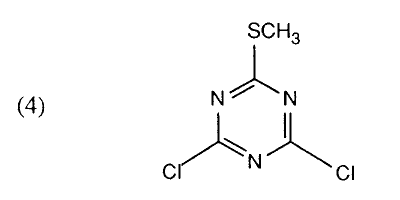 mittels einer Lewis-Säure mit m-Xylol oder Toluol umsetzt, in einer zweiten Stufe die erhaltene Verbindung der Formel (2) in Gegenwart von Toluol oder m-Xylol mit Chlor oder Sulfurylchlorid zur Verbindung der Formel (3) umsetzt und in einer dritten Stufe die erhaltene Verbindung mit 1,3-Dihydroxybenzol mittels einer Lewis-Säure zur Verbindung der Formel (1) in Gegenwart von Toluol, m-Xylol oder Xylol-Isomerengemisch umsetzt.

2. Verfahren gemäss Anspruch 1, dadurch gekennzeichnet, dass als Lewis-Säure Aluminiumchlorid verwendet wird.

3. Verfahren gemäss Anspruch 1 oder 2, dadurch gekennzeichnet, dass R₁ Wasserstoff bedeutet.

4. Verfahren gemäss Anspruch 1 oder 2, dadurch gekennzeichnet, dass R₁ Methyl bedeutet.

5. Verfahren gemäss Anspruch 1, dadurch gekennzeichnet, dass die Verbindungen der Formel (3) ohne Isolierung der Verbindungen der Formel (2) hergestellt werden.

6. Verfahren gemäss Anspruch 1, dadurch gekennzeichnet, dass die Reaktion der ersten Stufe zwischen 55 und 90°C, vorzugsweise zwischen 65 und 75°C, der zweiten Stufe zwischen 0 und 80°C, vorzugsweise zwischen 40 und 50°C und der dritten Stufe zwischen 60 und 100°C, vorzugsweise zwischen 80 und 90°C durchgeführt wird.

7. Verfahren zur Herstellung der Verbindungen der Formel (1) durch Umsetzung einer Verbindung der Formel (3) gemäss Anspruch 1 mit 1,3-Dihydroxybenzol mittels einer Lewis-Säure, dadurch gekennzeichnet, dass man die Reaktion in Gegenwart von Toluol, m-Xylol oder einem Xylol-Isomerengemisch durchführt.

8. Verbindung der Formel

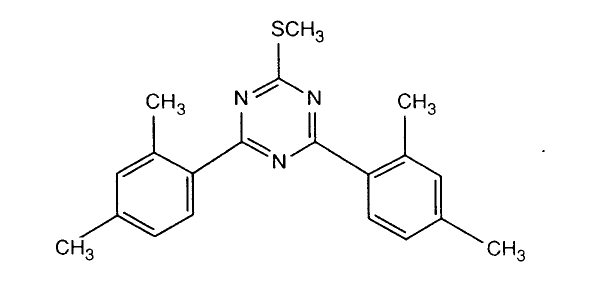

## Claims

1. A process for the preparation of a 2-(2',4'-dihydroxyphenyl)-4,6-diaryl-s-triazine of formula 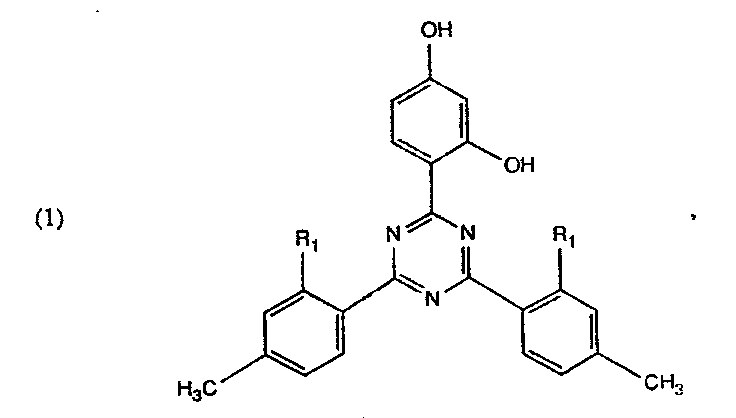 wherein R₁ is methyl or hydrogen, by replacing the methylthio group of a methylthio-diaryl-s-triazine of formula 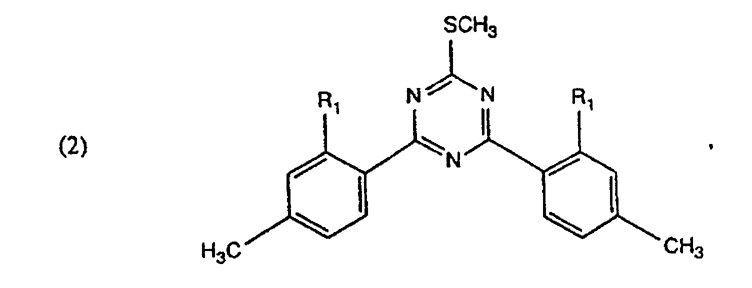 wherein R₁ is as defined above, by a chlorine atom and by reacting the resultant compound of formula 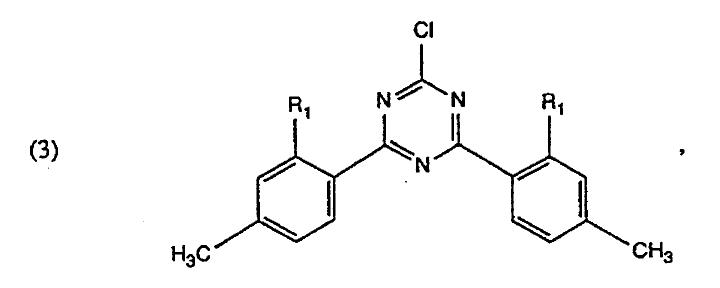 wherein R₁ is as defined above, with 1,3-dihydroxybenzene, with the aid of a Lewis acid, to give a compound of formula (1), which process comprises reacting, in a first step, the compound of formula 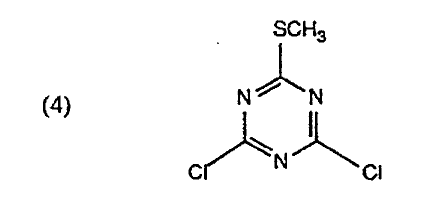 with the aid of a Lewis acid, with m-xylene or toluene, and, in a second step, reacting the resultant compound of formula (2), in the presence of toluene or m-xylene, with chlorine or sulfuryl chloride, to give the compound of formula (3), and, in a third step, reacting the resultant compound with 1,3-dihydroxybenzene, with the aid of a Lewis acid, to give the compound of formula (1), in the presence of toluene, m-xylene or a mixture of xylene isomers.

2. A process according to claim 1, wherein the Lewis acid used is aluminium chloride.

3. A process according to claim 1 or 2, wherein R₁ is hydrogen.

4. A process according to claim 1 or 2, wherein R₁ is methyl.

5. A process according to claim 1, wherein the compound of formula (3) is prepared without isolation of the compound of formula (2).

6. A process according to claim 1, wherein the reaction of the first step is carried out at between 55 and 90°C, preferably between 65 and 75°C, that of the second step at between 0 and 80°C, preferably between 40 and 50°C, and that of the third step at between 60 and 100°C, preferably between 80 and 90°C.

7. A process for the preparation of compounds of formula (1) by reacting a compound of formula (3) according to claim 1 with 1,3-dihydroxybenzene, with the aid of a Lewis acid, which process comprises carrying out the reaction in the presence of toluene, m-xylene or a mixture of xylene isomers.

8. A compound of formula

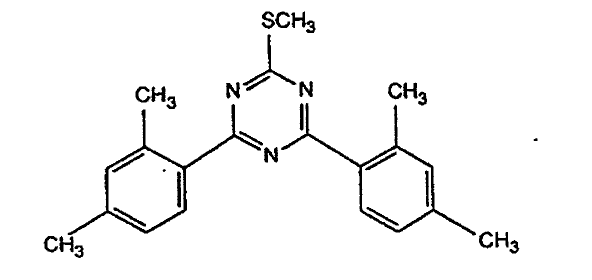

## Revendications

1. Procédé de préparation de 2-(2',4'-dihydroxyphényl)-4,6-diaryl-s-triazines, de formule 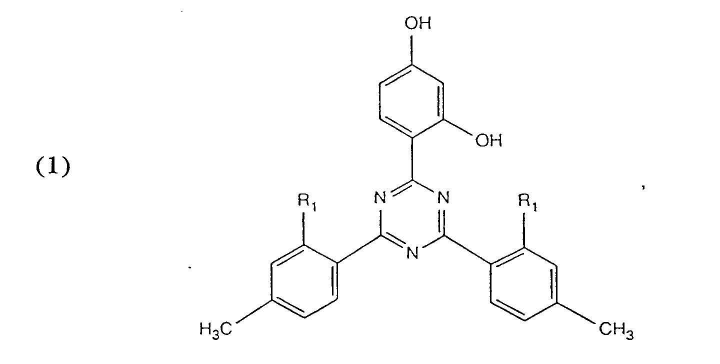 dans laquelle R₁ représente un groupe méthyle ou un atome d'hydrogène, par remplacement du groupe méthylthio d'une méthylthio-diaryl-s-triazine, de formule 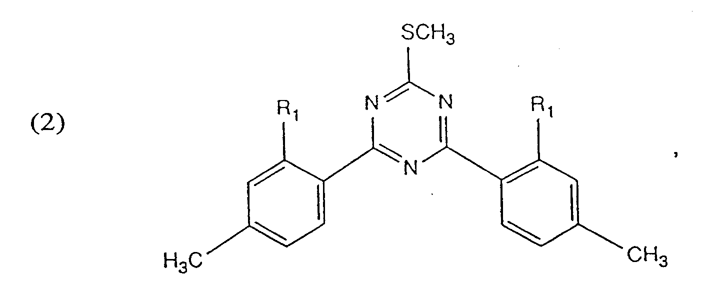 dans laquelle R₁ possède la signification indiquée ci-dessus, par un atome de chlore et réaction du composé obtenu, de formule 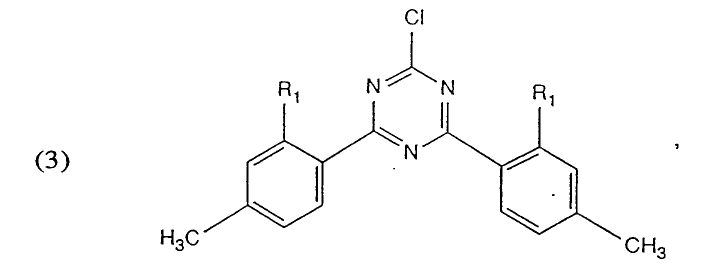 dans laquelle R₁ possède la signification indiquée ci-dessus, avec un 1,3-dihydroxybenzène, à l'aide d'un acide de Lewis, ce qui donne un composé de formule (1), procédé caractérisé en ce qu'on fait réagir, dans une première étape, le composé de formule 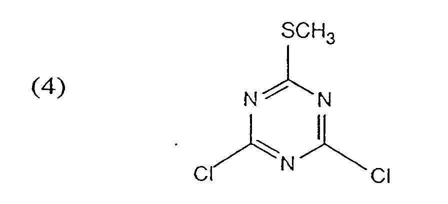 avec du toluène ou du m-xylène, à l'aide d'un acide de Lewis, puis on fait réagir, dans une seconde étape, le composé obtenu, de formule (2), avec du chlore ou du chlorure de sulfuryle, en présence de toluène ou de m-xylène, ce qui donne un composé de formule (3), et l'on fait réagir, dans une troisième étape, le composé ainsi obtenu avec du 1,3-dihydroxybenzène, à l'aide d'un acide de Lewis et en présence de toluène, de m-xylène ou d'un mélange d'isomères de xylène, ce qui donne un composé de formule (1).

2. Procédé conforme à la revendication 1, caractérisé on ce que l'on utilise du chlorure d'aluminium comme acide de Lewis.

3. Procédé conforme à la revendication 1 ou 2, caractérisé en ce que R₁ représente un atome d'hydrogène.

4. Procédé conforme à la revendication 1 ou 2, caractérisé en ce que R₁ représente un groupe méthyle.

5. Procédé conforme à la revendication 1, caractérisé en ce que l'on prépare les composés de formule (3) sans isoler les composés de formule (2).

6. Procédé conforme à la revendication 1, caractérisé en ce que l'on effectue la réaction de la première étape à une température située entre 55°C et 90°C, de préférence entre 65°C et 75°C, celle de la deuxième étape à une température située entre 0°C et 80°C, de préférence entre 40°C et 50°C, et celle de la troisième étape à une température située entre 60°C et 100°C, de préférence entre 80°C et 90°C.

7. Procédé de préparation de composés de formule (1), par réaction d'un composé de formule (3), indiquée dans la revendication 1, avec du 1,3-dihydroxybenzène, à l'aide d'un acide de Lewis, caractérisé en ce l'on effectue cette réaction en présence de toluène, de m-xylène ou d'un mélange d'isomères de xylène.

8. Composé de formule
